# EUROPEAN PATENT APPLICATION

(11) **EP 4 653 003 A1**
(43) Date of publication of application: **26.11.2025**
(21) Application number: 24744692.5
(22) Date of filing: 18.01.2024
(51) Int. Cl.: A61K 33/244, A61P 13/12

(54) **PHARMACEUTICAL COMPOSITION HAVING CERIUM COMPOUND CONTAINING TRIVALENT CERIUM AS ACTIVE INGREDIENT**

(30) Priority: 18.01.2023 WO PCT/JP2023/001415
(71) Applicant: Applause Pharma Co., Ltd., Tokyo 104-0033 (JP); Mitsui Mining & Smelting Co., Ltd., Shinagawa-ku Tokyo 141-8584 (JP)
(72) Inventor: KAWAGUCHI, Tsunetaka, Tokyo 104-0033 (JP); NAKATSU, Masaharu, Tokyo 104-0033 (JP); OGAWA, Yukihiro, Tokyo 104-0033 (JP)
(74) Representative: Witthoff Jaekel Steinecke Patentanwälte PartG mbB
(86) International application number: PCT/JP2024/001237
(87) International publication number: WO 2024/154771

(57) **Abstract**

A first object of the present invention is to provide a novel pharmaceutical composition that contains a cerium compound as an active ingredient. It is therefore a second object of the present invention to provide a therapeutic agent for improvement of renal function, particularly to provide a pharmaceutical composition for lowering, maintaining, or suppressing elevation of at least one test value selected from serum creatinine (CRE), blood urea nitrogen (BUN), and serum phosphorus (IP).

The present invention aimed at solving the aforementioned problem relates to a pharmaceutical composition having, as an active ingredient, a cerium compound that contains trivalent cerium.

## Description

### Technical Field

The present invention relates to a pharmaceutical composition having, as an active ingredient, a cerium compound that contains trivalent cerium.

The present invention also relates to a renal function improving drug, particularly a renal function improving drug for use in lowering, maintenance, or suppression of elevation of at least one test value selected from serum creatinine (CRE), blood urea nitrogen (BUN), or blood phosphorus (IP).

### Background Art

Kidney disease involves damages typically on renal glomerulus and tubule to cause renal dysfunction, for which various types have been known. In terms of pathological condition, the kidney disease will progress to lead to renal function declination, or a status called renal failure. The renal failure includes acute renal failure typified by sharp decline of renal function, and chronic renal failure typified by decline of renal function over a long term. The acute renal failure would be cured, if the cause of worsening the renal function could be eliminated. For the chronic renal failure, there is, however, little hope for recovering the renal function having been lost due to progress of renal failure.

A condition demonstrating a uremic symptom due to accumulated uremic toxin in the body ascribed to renal failure, for which dialysis is however not yet necessary, is referred to as conservative management of renal failure. In this period, the progress of renal failure is delayed by blood pressure management with use of antihypertensive drug; dietary therapies such as restriction of water intake, and restriction of intake of protein, phosphorus or potassium; and drug therapy adapted to the symptom.

Further progress of the renal function declination from the conservative management of renal failure will lead to end stage renal failure (dialysis period). The end stage renal failure requires treatment for removing waste products accumulated in the body, for which dialysis therapy (peritoneal dialysis or hemodialysis) will be required as a symptomatic therapy, while kidney transplantation (deceased donor kidney transplantation or living kidney transplantation) will be required as a radical therapy.

All chronically progressing kidney disease are referred to as chronic kidney disease (CKD), advocated in the United States in 2002. The chronic kidney disease (CKD) is not only a risk factor for progression to the end stage renal failure, but also a risk factor for onset of cardiovascular disease.

Improvement of the chronic kidney disease (CKD) has employed antihypertensive drug, diuretic, insulin preparation, statin-based drug, fibrate-based drug, small intestinal cholesterol transporter inhibitor, erythropoietin preparation, acidosis therapeutic agent, phosphorus adsorbent, vitamin D3 preparation, potassium adsorbent, carbon adsorbent preparation, steroids/immunosuppressant, Chinese medicine or the like, having been selected depending on causes of renal failure, or conditions of the patient.

None of these drugs, however, directly improves the renal function, instead being intended for symptomatic therapy or adjuvant therapy so as to delay the progress of the disease state or to prevent complications. There is no radical drug for chronic kidney disease (CKD) at present.

WO 2022/014122 A1 discloses that a cerium oxide microparticle, as a pharmaceutical composition that contains a cerium compound as an active ingredient, can lower levels of serum creatinine (CRE), blood urea nitrogen (BUN), and blood phosphorus (IP) in a nephropathy model system, without increasing blood level of alanine aminotransaminase (ALT), and is proven to be effective as a drug for improving renal function (Patent Literature 1).

Note now cerium nitrate, which is a kind of cerium salt, has been used typically in the form of wound healing drug in combination with silver sulfadiazine, for example in Belgium, the Netherlands, France, Spain, and the United Kingdom, as a topical treatment for burn.

Cerium oxalate has been reported to be effective when applied to "nausea and vomiting due to hyperemesis gravidarum or motion sickness", but not to be effective as a renal function improving drug.

In Japan, cerium oxalate has been judged to "have no evidence of efficacy", since other appropriate drug or therapy is available.

### Citation List

### Patent Literature

Patent Literature 1: WO 2022/014122 A1

### Summary of Invention

### Technical Problem

As described previously, as for the pharmaceutical composition that contains, as an active ingredient, a cerium compound, cerium oxide has been known as an ingredient proven in vivo to be effective in improving renal function, in terms of test values of representative items for evaluating renal function, such as serum creatinine (CRE), blood urea nitrogen (BUN), or blood phosphorus (IP). Cerium oxide, however, has issues on stability during production, or temporal shelf stability, in order to enhance the ability for improving renal function, or to stabilize the performance of the fine particle.

On the other hand, cerium nitrate and cerium oxalate have not been proven to be effective as drugs for improving renal function.

Considering the situation, it is therefore a first object of the present invention to provide a novel pharmaceutical composition that contains a cerium compound as an active ingredient.

It is therefore a second object of the present invention to provide a therapeutic agent for improvement of renal function, particularly to provide a pharmaceutical composition for lowering, maintaining, or suppressing elevation of at least one test value selected from serum creatinine (CRE), blood urea nitrogen (BUN), and serum phosphorus (IP).

It is therefore a third object of the present invention to provide a pharmaceutical composition intended for use in lowering, maintenance, or suppression of elevation of blood uremic toxin level.

### Solution to Problem

The present invention aimed at solving the aforementioned problem relates to a pharmaceutical composition having, as an active ingredient, a cerium compound that contains trivalent cerium.

In a preferred mode of the present invention, the pharmaceutical composition is used for improving renal function.

In a preferred mode of the present invention, the cerium compound is one of, or two or more species selected from cerium chloride, cerium nitrate, cerium acetate, and hydrates of these compounds.

In a preferred mode of the present invention, the pharmaceutical composition of the present invention contains cerium oxide.

In a preferred mode of the present invention, the pharmaceutical composition is intended for use in lowering, maintenance, or suppression of elevation of at least one test value selected from serum creatinine (CRE), blood urea nitrogen (BUN), or blood phosphorus (IP).

In a preferred mode of the present invention, the pharmaceutical composition is intended for use in lowering, maintenance, or suppression of elevation of uremic toxin level.

In a preferred mode of the present invention, the uremic toxin is blood uremic toxin.

In a preferred mode of the present invention, the uremic toxin is any one of, or two or more species selected from indoxyl sulfate, p-cresyl sulfate, phenyl sulfate, and trimethylamine-N-oxide (TMAO).

In a preferred mode of the present invention, the cerium compound is an ionic compound.

In a preferred mode of the present invention, the cerium compound is one of, or two or more species selected from cerium chloride, cerium nitrate, cerium acetate, cerium citrate, and hydrates of these compounds.

In a preferred mode of the present invention, the pharmaceutical composition is intended for use in improvement of renal function, and/or treatment, suppression or improvement of uremia.

In a preferred mode of the present invention, the pharmaceutical composition of the present invention is gastrointestinally absorbable.

The present invention of this mode demonstrates excellent effects typically in lowering serum creatinine (CRE), blood urea nitrogen (BUN) level and/or serum phosphorus (IP) level, and/or in lowering uremic toxin level, as a result of gastrointestinal absorption of cerium, which is an active ingredient, into the body.

The present invention for solving the aforementioned problems relates to the followings.
[1] A pharmaceutical composition having, as an active ingredient, a cerium compound that contains trivalent cerium.
[2] The pharmaceutical composition according to [1], wherein the cerium compound is an ionic compound.
[3] The pharmaceutical composition according to [1] or [2], intended for use in improvement of renal function, and/or treatment, suppression or improvement of uremia.
[4] The pharmaceutical composition according to any one of [1] to [3], wherein the cerium compound is any one of, or two or more species selected from cerium chloride, cerium acetate, cerium nitrate, and hydrates of these compounds.
[5] The pharmaceutical composition according to any one of [1] to [4], containing cerium oxide.
[6] The pharmaceutical composition according to any one of [1] to [5], intended for use in lowering, maintenance, or suppression of elevation of at least one test value selected from serum creatinine (CRE), blood urea nitrogen (BUN), or blood phosphorus (IP).
[7] The pharmaceutical composition according to any one of [1] to [6], intended for use in lowering, maintenance, or suppression of elevation of uremic toxin level.
[8] The pharmaceutical composition according to [7], wherein the uremic toxin is blood uremic toxin.
[9] The pharmaceutical composition according to [7] or [8], wherein the uremic toxin is any one of, or two or more species selected from indoxyl sulfate, p-cresyl sulfate, phenyl sulfate, and trimethylamine-N-oxide (TMAO).
[10] The pharmaceutical composition according to any one of [1] to [9], being gastrointestinally absorbable.

The present invention may further be implemented in the modes below. Note that the preferred modes are as described above.
(1) Use of a cerium compound that contains trivalent cerium, for the manufacture of a pharmaceutical composition intended for improvement of renal function, and/or for treatment, suppression or improvement of uremia.
(2) Use of a cerium compound that contains trivalent cerium, as an active ingredient intended for improvement of renal function, and/or for treatment, suppression or improvement of uremia.
(3) A cerium compound that contains trivalent cerium, for use in improvement of renal function, and/or for treatment, suppression or improvement of uremia.
(4) A method for improving renal function, and/or for treating, suppressing or improving uremia, the method including administering a cerium compound that contains trivalent cerium, to a subject in need of improvement in renal function, and/or in need of treatment, suppression or improvement of uremia.

### Advantageous Effects of Invention

The pharmaceutical composition of the present invention, having as an active ingredient, a cerium compound that contains trivalent cerium, is effective typically in lowering, maintenance, or suppression of elevation of serum creatinine (CRE), blood urea nitrogen (BUN), and/or blood phosphorus (IP), which are values of test items for evaluating renal function.

The pharmaceutical composition of the present invention having, as an active ingredient, a cerium compound that contains trivalent cerium, is also effective for lowering, maintenance, or suppression of elevation of uremic toxin level in the body, particularly in blood.

### Brief Description of Drawings

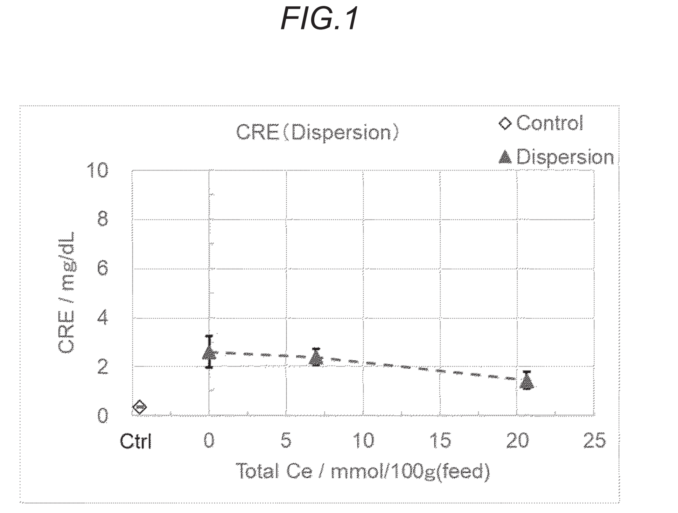

Fig. 1 is a diagram illustrating serum creatinine levels (CRE) under normal feed (control), under adenine feed (total cerium = 0 mmol/100 g feed), and under adenine + cerium oxide dispersion feed. The diagram teaches that the CRE level declines depending on the total cerium (mol) in 100 g feed.

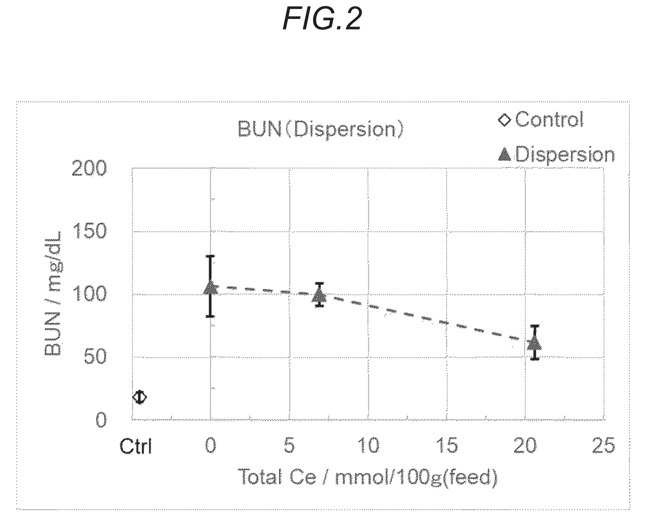

Fig. 2 is a diagram illustrating blood urea nitrogen levels (BUN) under normal feed (control), under adenine feed (total cerium = 0 mmol/100 g feed), and under the adenine + cerium oxide dispersion feed. The diagram teaches that the BUN level declines depending on the total cerium content (mol) in 100 g feed.

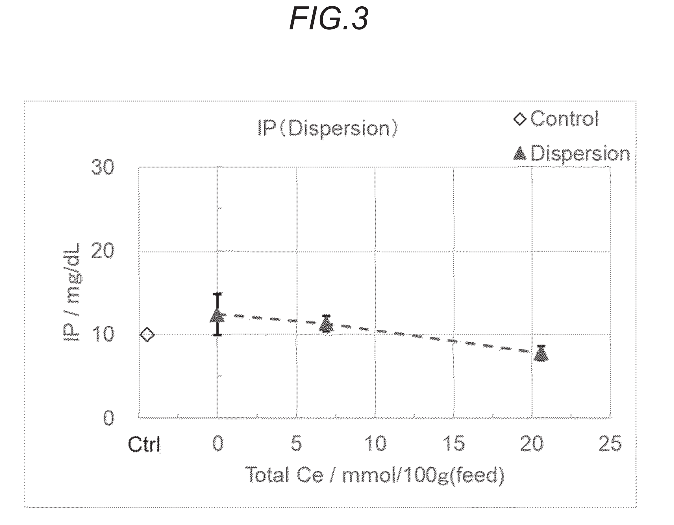

Fig. 3 is a graph illustrating blood phosphorus levels (IP) under normal feed (control), under adenine feed (total cerium = 0 mmol/100 g feed), and under adenine + cerium oxide dispersion feed. The diagram teaches that the IP level declines depending on the total cerium content (mol) in 100 g feed.

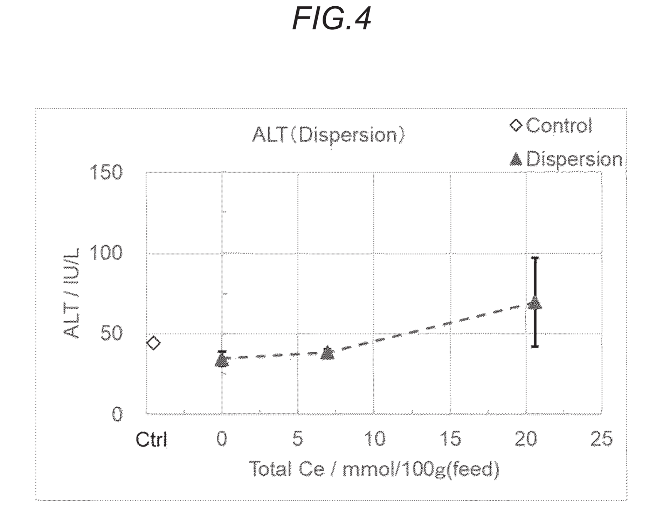

Fig. 4 is a diagram illustrating alanine aminotransferase levels (ALT) under normal feed (control), under adenine feed (total cerium = 0 mmol/100 g feed), and under adenine + cerium oxide dispersion feed. The diagram illustrates relation between the total cerium content (mol) in 100 g feed, and ALT.

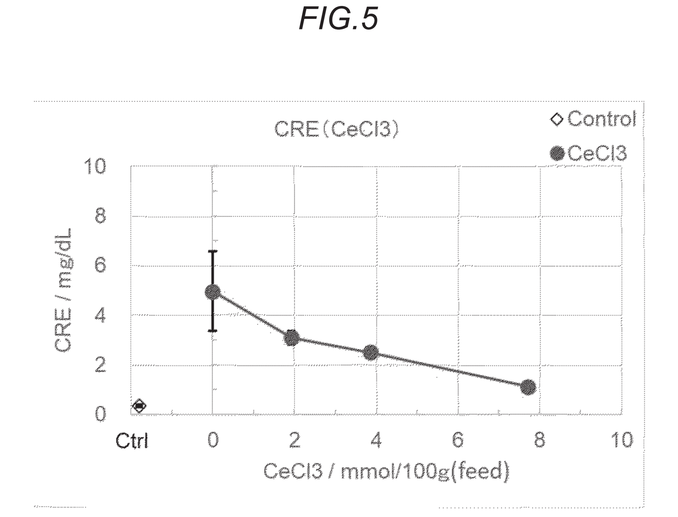

Fig. 5 is a diagram illustrating serum creatinine levels (CRE) under normal feed (control), under adenine feed (CeCl₃ = 0 mmol/100 g feed), and under adenine + cerium chloride feed. The diagram teaches that the CRE level declines depending on the cerium chloride content (mol) in 100 g feed.

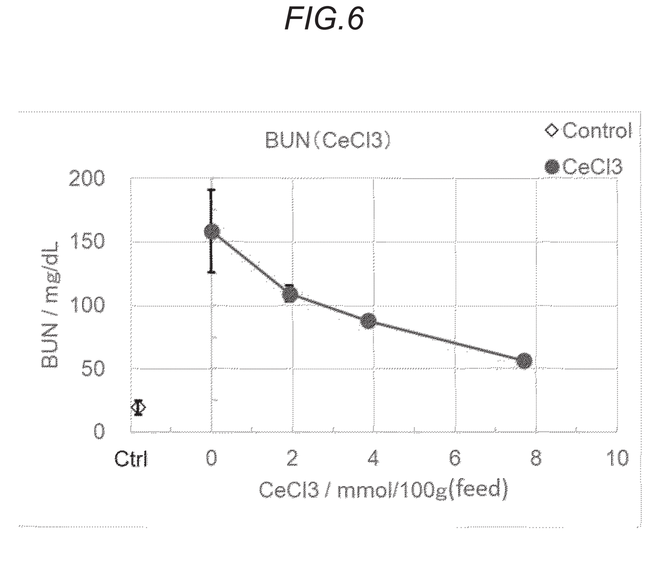

Fig. 6 is a diagram illustrating blood urea nitrogen levels (BUN) under normal feed (control), under adenine feed (CeCl₃ = 0 mmol/100 g feed), and under adenine + cerium chloride feed. The diagram teaches that the BUN level declines depending on the cerium chloride content (mol) in 100 g feed.

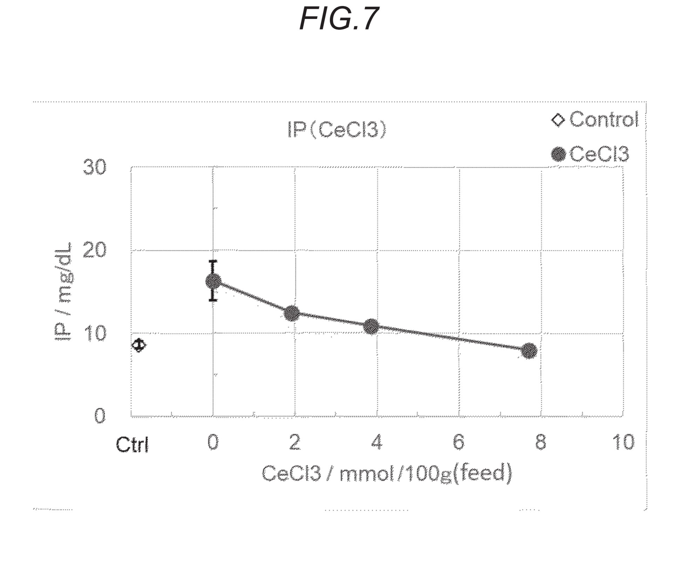

Fig. 7 is a diagram illustrating blood phosphorus levels (IP) under normal feed (control), under adenine feed (CeCl₃ = 0 mmol/100 g feed), and under adenine + cerium chloride feed. The diagram teaches that the IP level declines depending on the cerium chloride content (mol) in 100 g feed.

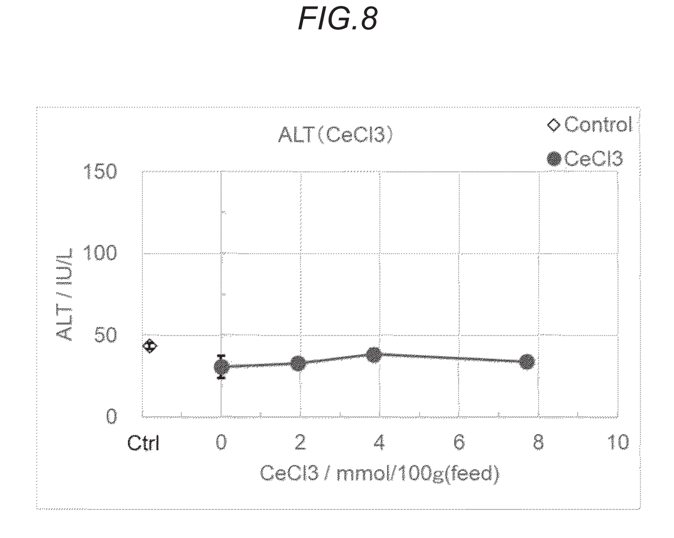

Fig. 8 is a diagram illustrating alanine aminotransferase levels (ALT) under normal feed (control), under adenine feed (CeCl₃ = 0 mmol/100 g feed), and under adenine + cerium chloride feed. The diagram illustrates relation between the cerium chloride content (mol) in 100 g of feed, and ALT.

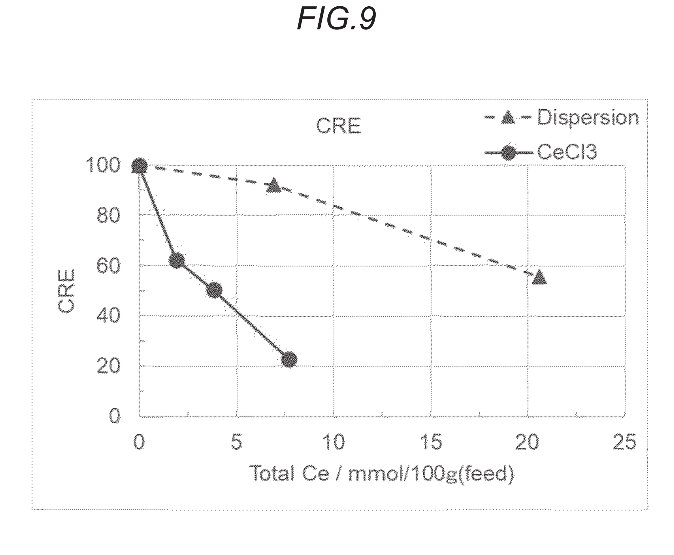

Fig. 9 is a diagram comparatively illustrating serum creatinine levels (CRE) relative to the total molar amount of cerium in cerium oxide dispersion, and relative to the molar amount of cerium in CeCl₃, normalized while scaling an averaged serum creatinine level (CRE) in an adenine feed group at 100.

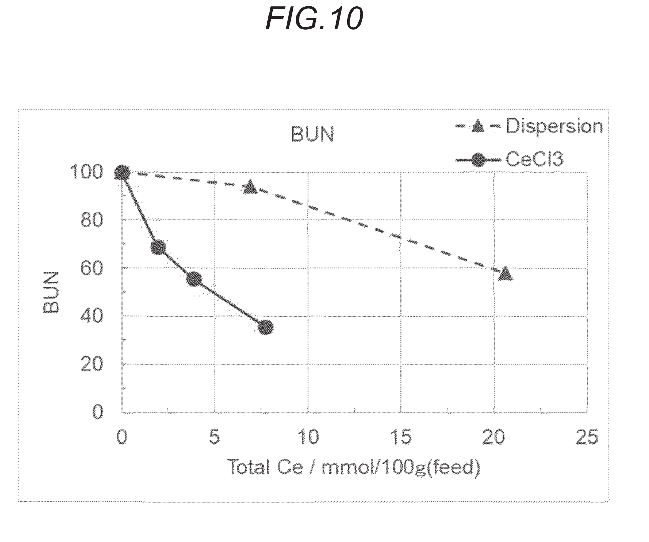

Fig. 10 is a diagram comparatively illustrating blood urea nitrogen levels (BUN) relative to the total molar amount of cerium in cerium oxide dispersion, and relative to the molar amount of cerium in CeCl₃, normalized while scaling an averaged blood urea nitrogen level (BUN) in the adenine feed group at 100.

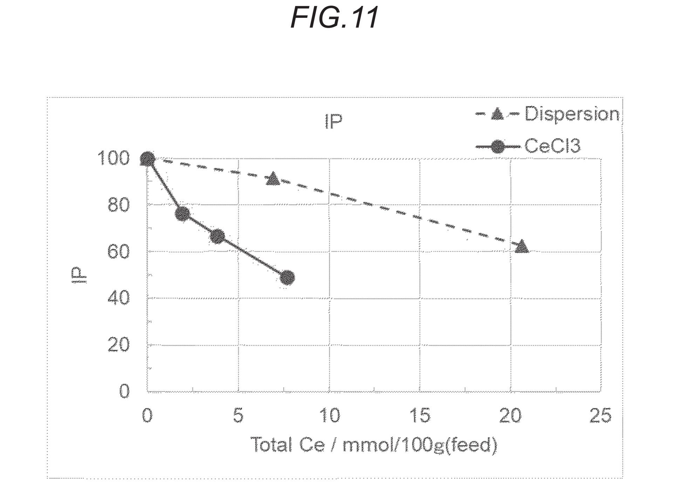

Fig. 11 is a diagram comparatively illustrating blood phosphorus levels (IP) relative to the total molar amount of cerium in cerium oxide dispersion, and relative to the molar amount of cerium in CeCl₃, normalized while scaling an averaged blood phosphorus level (IP) in the adenine feed group at 100.

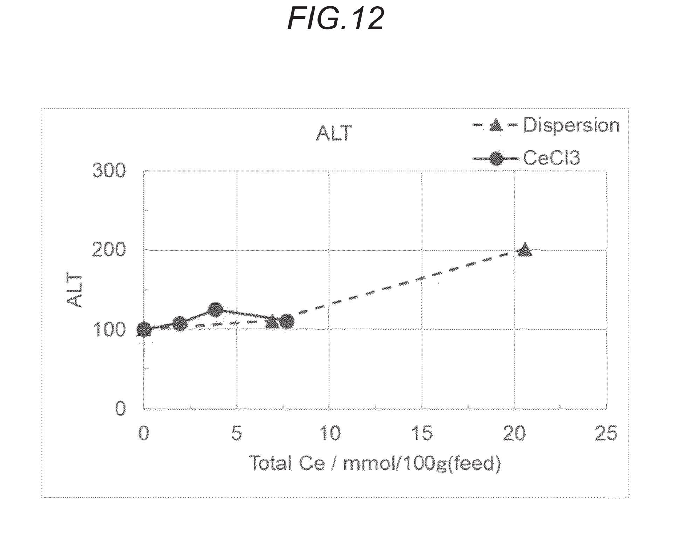

Fig. 12 is a diagram comparatively illustrating alanine aminotransferase level (ALT) relative to the total molar amount of cerium in cerium oxide dispersion, and relative to the molar amount of cerium in CeCl₃, normalized while scaling an averaged alanine aminotransferase level (ALT) in the adenine feed group at 100.

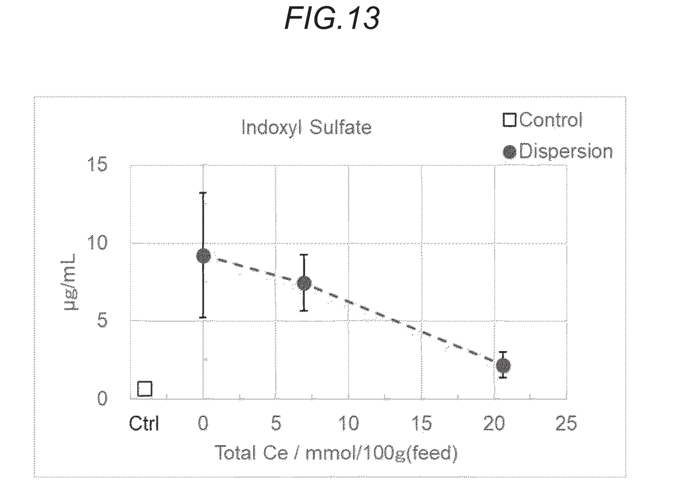

Fig. 13 is a diagram illustrating plasma indoxyl sulfate levels under normal feed (control), under adenine feed (total cerium content = 0 mmol/100 g feed), and under adenine + cerium oxide dispersion feed. The diagram teaches that the plasma indoxyl sulfate level declines depending on the total cerium content (mol) in 100 g feed.

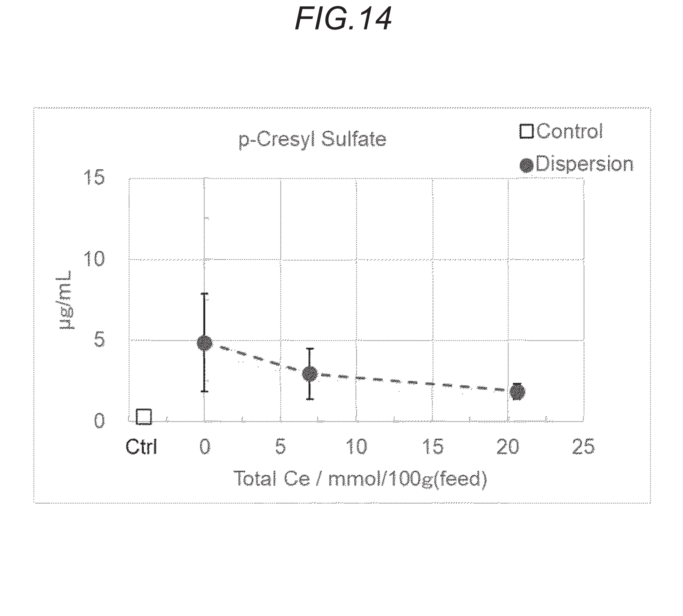

Fig. 14 is a diagram illustrating plasma p-cresyl sulfate levels under normal feed (control), under adenine feed (total cerium content = 0 mmol/100 g feed), and under adenine + cerium oxide dispersion feed. The diagram teaches that the plasma p-cresyl sulfate level declines depending on the total cerium content (mol) in 100 g feed.

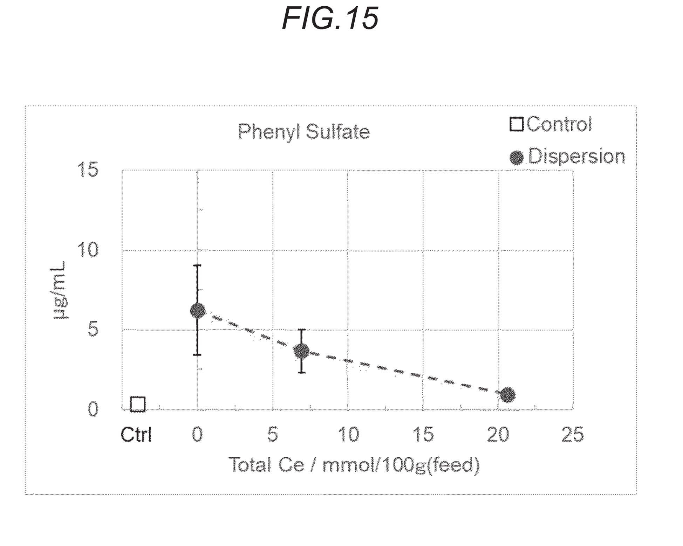

Fig. 15 is a diagram illustrating plasma phenyl sulfate levels under normal feed (control), under adenine feed (total cerium content = 0 mmol/100 g feed), and under adenine + cerium oxide dispersion feed. The diagram teaches that the plasma phenyl sulfate level declines depending on the total cerium content (mol) in 100 g feed.

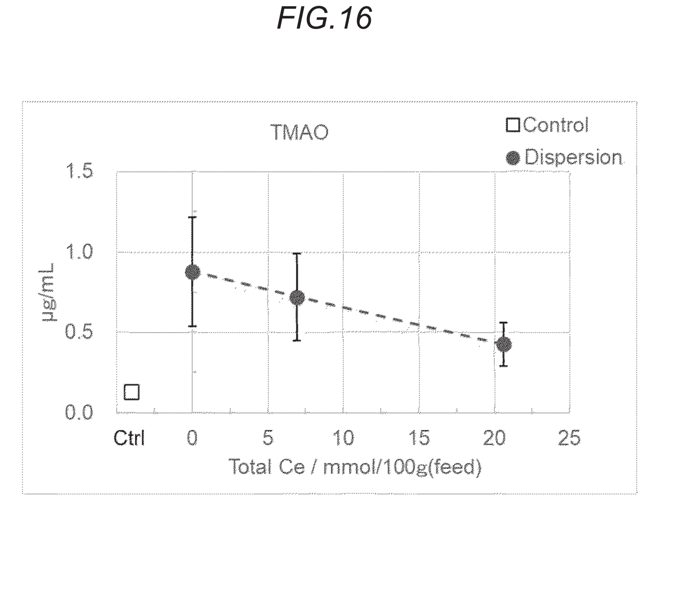

Fig. 16 is a diagram illustrating plasma trimethylamine-N-oxide (TMAO) levels under normal feed (control), under adenine feed (total cerium content = 0 mmol/100 g feed), and under adenine + cerium oxide dispersion feed. The diagram teaches that the plasma trimethylamine-N-oxide (TMAO) level declines depending on the total cerium content (mol) in 100 g feed.

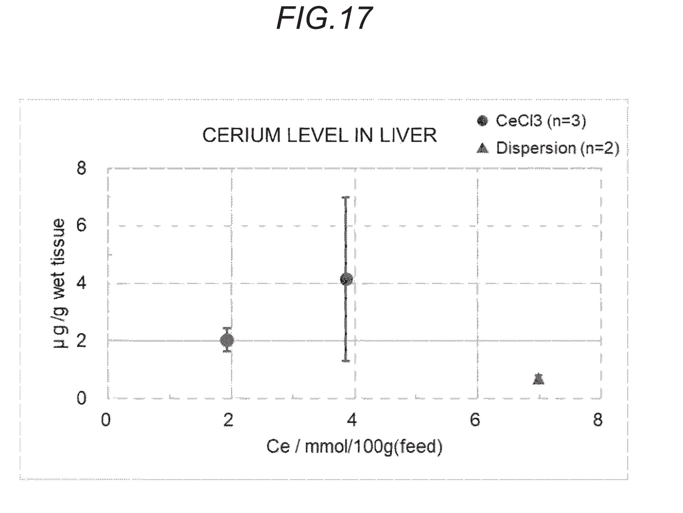

Fig. 17 is a diagram illustrating cerium levels detected from livers under cerium oxide dispersion liquid feed, and under the adenine + cerium chloride mixed feed. The diagram teaches that, under the adenine + cerium chloride mixed feed, the cerium level detected from the liver increases depending on the total cerium content (mol) in 100 g feed.

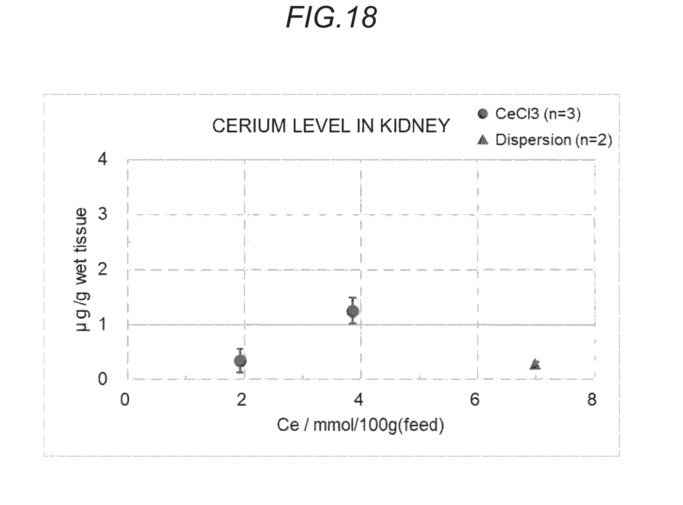

Fig. 18 is a diagram illustrating cerium levels detected from kidneys under cerium oxide dispersion liquid feed, and adenine + cerium chloride mixed feed. The diagram teaches that, under the adenine + cerium chloride mixed feed, the cerium level detected from the kidneys increases depending on the total cerium content (mol) in 100 g feed.

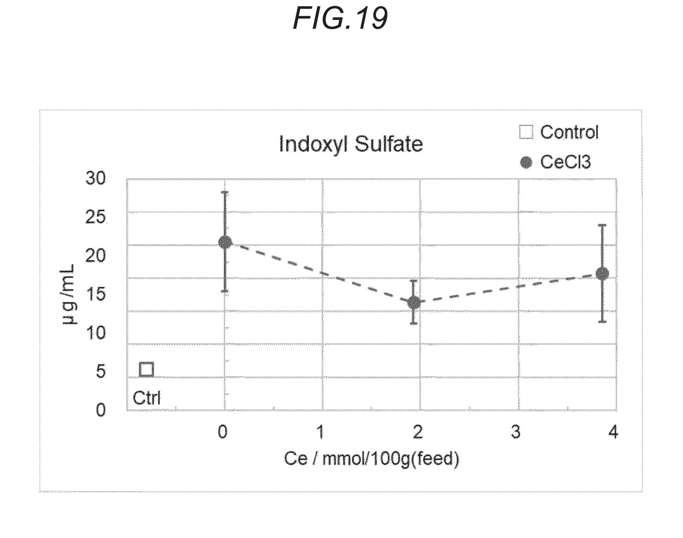

Fig. 19 is a diagram illustrating indoxyl sulfate levels in whole blood under normal feed (control), under adenine feed (total cerium content = 0 mmol/100 g feed), and under adenine + cerium chloride mixed feed.

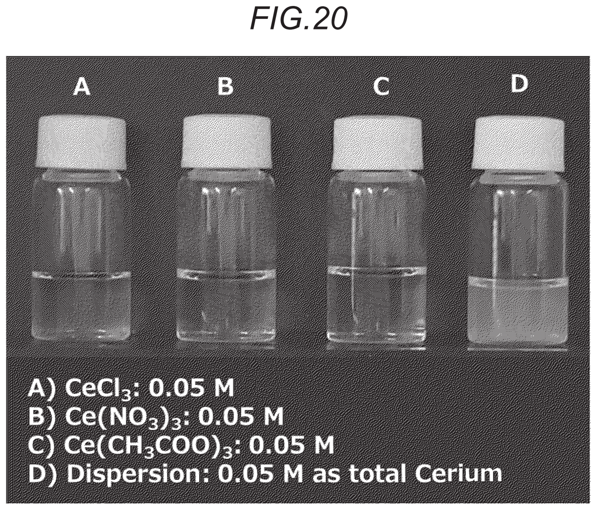

Fig. 20 is a diagram illustrating appearances of simulated gastric fluids each having cerium chloride, cerium nitrate, cerium acetate, or cerium oxide dispersion added thereto, while adjusting the total cerium content in fluid to 0.05 M.

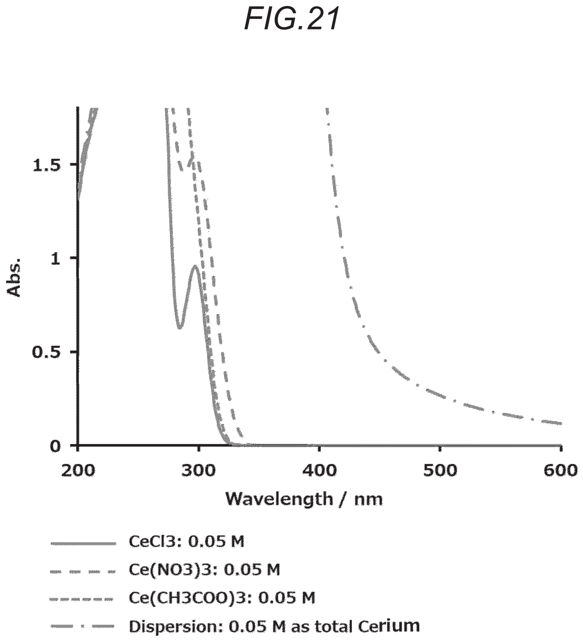

Fig. 21 is a diagram illustrating absorption spectra of the simulated gastric fluids each having cerium chloride, cerium nitrate, cerium acetate, or cerium oxide dispersion added thereto while adjusting the total cerium content in fluid to 0.05 M.

### Description of Embodiments

Next, preferred embodiments of the present invention will be detailed. Note that the present invention is not limited to the following embodiments, and is freely modifiable within the scope of the present invention.

The pharmaceutical composition of the present invention has, as the active ingredient, the cerium compound that contains trivalent cerium. Now, the pharmaceutical composition of the present invention encompasses not only a mode such that the cerium compound per se functions as the active ingredient, but also a mode such that trivalent cerium ion released from the cerium compound functions as the active ingredient.

### (1) Cerium Compound

The cerium compound in the present invention is preferably metal salt or complex that contains trivalent cerium, or hydrate thereof. The cerium compound is exemplified by cerium(III) nitrate, cerium(III) chloride, cerium(III) bromide, cerium(III) iodide, cerium(III) perchlorate, cerium(III) iodate, cerium(III) sulfate, cerium(III) carbonate, cerium(III) phosphate, cerium(III) ammonium nitrate, cerium(III) formate, cerium(III) acetate, cerium(III) propionate, cerium(III) butyrate, cerium(III) pentanoate, cerium(III) hexanoate, cerium(III) octanoate, cerium(III) oleate, cerium(III) lactate, cerium(III) citrate, cerium(III) oxalate, cerium(III) phosphate, cerium(III)-EDTA, and, hydrates of these compounds.

Only one kind of these cerium compounds may be used singly, or two or more kinds may be used in a combined manner.

In a preferred mode of the present invention, the cerium compound in the present invention is an ionic compound. The ionic compound is preferably exemplified by metal salt that contains trivalent cerium, and hydrate thereof.

The ionic compound in the present invention dissolves in the gastrointestinal tract after ingested, and gastrointestinally absorbed inside the body, thereby exerting an effect as a therapeutic agent for kidney disease or an application for lowering uremic toxin.

Note that "inside the body" in the context of the present invention means the inner side of the body reached after permeation through the gastrointestinal wall, meanwhile the outer side of the gastrointestinal wall (the space surrounded by the gastrointestinal wall) is defined as "outside the body".

The cerium compound suitably used in the present invention may be one, or two or more species selected from cerium(III) chloride, cerium(III) nitrate, cerium(III) acetate, and hydrates of these compounds. More specifically, the cerium compounds, with the hydration numbers rounded off to the first decimal place, are suitably exemplified by cerium(III) chloride anhydride or hydrates thereof (monohydrate, dihydrate, trihydrate, tetrahydrate, pentahydrate, hexahydrate, heptahydrate, octahydrate, nonahydrate, and decahydrate); cerium(III) nitrate anhydride or hydrates thereof (dihydrate, trihydrate, tetrahydrate, pentahydrate, hexahydrate, heptahydrate, octahydrate, nonahydrate, decahydrate, and dodecahydrate); and cerium(III) acetate anhydrate or hydrates thereof (monohydrate, dihydrate, trihydrate, tetrahydrate, pentahydrate, and hexahydrate). In the present invention, cerium(III) chloride hexahydrate, heptahydrate or octahydrate is preferably used, among which cerium(III) chloride heptahydrate is further preferably used. Also use of cerium(III) acetate anhydrate or hydrate thereof is exemplified as a suitable mode.

The cerium compound used in the present invention may be one, or two or more species selected from cerium(III) chloride, cerium(III) nitrate, cerium(III) acetate, cerium(III) citrate, and hydrates of these compounds.

The content of the cerium compound as the active ingredient of the present invention, relative to the total amount of the pharmaceutical composition of the present invention, is preferably 0.001 to 100% by mass, more preferably 0.003 to 99% by mass, and even more preferably 0.01 to 98% by mass.

### (2) Other Components

The pharmaceutical composition of the present invention may optionally contain, besides the cerium compound that contains trivalent cerium, any additive that can be blended to a pharmaceutical product, so far as the purpose of the present invention may be accomplished.

The pharmaceutical composition of the present invention may be in a form that contains cerium oxide and a hydrate thereof.

The cerium oxide in the present invention may be either cerium(III) oxide or cerium(IV) oxide, which is preferably cerium(IV) oxide.

The cerium oxide is preferably in the form of particle, and more preferably in the form of fine particle. Average primary particle size of the particle is 100 nm or smaller, preferably 50 nm or smaller, more preferably 20 nm or smaller, even more preferably 10 nm or smaller, and most preferably 5 nm or smaller.

With such small particle size, the cerium oxide used herein will have large total surface area, that is, a large area interactive with a target substance such as adsorbate, making it possible to provide a pharmaceutical composition with more advanced action.

Now, the average primary particle size may be estimated by taking an image of the particles under an electron microscope, measuring the diameter of ten or more randomly selected particles on the captured image, and averaging values of the measured diameter. The estimation may alternatively rely upon scattering spectral analysis based on dynamic light scattering, wherein correction by observation under an electron microscope will be necessary.

The cerium oxide blendable to the pharmaceutical composition of the present invention is preferably coated with a dispersant.

With such coating with the dispersant, the cerium oxide particles will be suppressed from mutually aggregating. Accordingly, the particle will have large total surface area, that is, a large area interactive with a target substance such as adsorbate, making it possible to provide a pharmaceutical composition with more advanced action.

Examples of the dispersant that coats cerium oxide include acids and salts of acids, such as sodium acetate, ammonium acetate, sodium propionate, ammonium propionate, sodium octanoate, ammonium octanoate, lysine, histidine, proline, threonine, isoleucine, alanine, leucine, glycine, valine, methionine, serine, tyrosine, glutamine, asparagine, cysteine, 3-aminopropanoic acid, 4-aminobutyric acid, 5-aminopentanoic acid, 6-aminohexanoic acid, EDTAs, succinic acid, lactic acid, citric acid, oleic acid, and metaphosphoric acid.

One species of, or combination of two or more species selected from these dispersants, may be used.

Amount of the dispersant that coats cerium oxide, blendable in the pharmaceutical composition of the present invention, is 1:0.001 to 1:10 in molar ratio relative to cerium oxide, which is preferably 1:0.01 to 1:5, and is more preferably 1:0.02 to 1:2.

In a case where two or more species of dispersant are used, the molar ratio of the total content of the dispersants to the cerium oxide preferably falls within any of the aforementioned ranges.

Cerium oxide used in the present invention is preferably dispersed in a base material such as water, thereby given in the form of cerium oxide dispersion. The aqueous cerium oxide dispersion may be prepared by the method described in WO 2022/014122 A1.

Other optional components that can be blended in the pharmaceutical composition of the present invention include complexing agent, excipient, lubricant, fluidizing agent, binder, disintegrant, stabilizer, preservative, buffer, flavoring agent, sweetener, suspending agent, emulsifier, fragrance, solubilizing agent, colorant, thickener, various nutrients, vitamins, and antioxidant, all of which may be added singly or in combination of any two or more species.

Examples of the blendable substance include acids and salts of acids, such as sodium acetate, ammonium acetate, sodium propionate, ammonium propionate, sodium octanoate, ammonium octanoate, lysine, histidine, proline, threonine, isoleucine, alanine, leucine, glycine, valine, methionine, serine, tyrosine, glutamine, asparagine, cysteine, 3-aminopropanoic acid, 4-aminobutyric acid, 5-aminopentanoic acid, 6-aminohexanoic acid, EDTAs, succinic acid, lactic acid, citric acid, oleic acid, metaphosphoric acid, ascorbic acid, malic acid, tartaric acid, glucuronic acid, gallic acid, glyceric acid, hydroxybutyric acid and glyoxylic acid; lactose; crystalline cellulose; starch; agar; gelatin; dry syrup; sugars (monosaccharide, disaccharide, oligosaccharide, or polysaccharide); electrolyte; and dietary fiber.

The pharmaceutical composition of the present invention, when dispersed or dissolved in water, preferably exhibits a pH of 1 to 10, more preferably 2 to 9, and even more preferably 3 to 8.

### (3) Dosage Form

The pharmaceutical composition of the present invention may be used in a dosage form of oral preparation, injection, or external preparation.

Examples of the oral preparation include tablets (including tablet with ordinary coating, dragee, sublingual tablet, and buccal tablet), capsule, granule, powder, fine granule, syrup (including dry syrup), enteric coating preparation (including enteric capsule), sustained-release capsule, cachet (including oblate pouch), chewable tablet, drop, pill, liquid for internal use (including all liquid dosage forms orally administered), candy tablet (troche, candies, etc.), sustained-release tablet, and sustained-release granule.

Examples of the injection include those dissolved at the time of use, and those also containing total parenteral nutrient.

Examples of the external preparation include a dusting powder, lotion, ointment/cream, shampoo, spray (excluding inhalant), external liquid preparation (including liniment), tape preparation (including poultice), aerosol (metered-dose spray aerosol for inhalation), ear drop preparation, eye drop, eye drop ointment, nasal drop (including nasal spray), gargle preparation, anal suppository, insertion preparation (vaginal suppository, vaginal tablet, etc.), enema preparation, jelly, and peritoneal perfusate.

The pharmaceutical composition of the present invention can also be used for infusion fluid or drug delivery system (DDS) preparation.

Packaging of the pharmaceutical composition of the present invention is not particularly limited, as long as it is suitable for ensuring quality of the pharmaceutical composition, proper use, and ensuring safety at the time of administration. The packaging is designed taking information on manufacturing process of a packaging material into consideration, in order to verify eligibility of packaging form and packaging material to be selected, and to maintain eligibility of pharmaceutical packaging throughout lifecycle of the product.

### (4) Use, and Target of Administration

Representative test items for determining renal function include serum creatinine (CRE) and urea nitrogen (BUN) .

Creatine phosphate, which is an important energy source for muscle to move, is metabolized to produce creatinine as a waste. Although creatinine is filtered in the kidneys to be excreted in urine, an unfiltered portion will remain in the blood, if the renal function declines. It is therefore understood that the higher the blood creatinine level, the lower the renal function.

Proteins are consumed to produce ammonia, and the toxic ammonia is converted to urea in the liver. Urea nitrogen is a nitrogen component contained in blood urea, and is excreted in urine after filtered through the kidneys. An unfiltered portion will, however, remain in the blood, if the renal function declines. It is therefore understood that the higher the blood urea nitrogen level, the lower the renal function.

Phosphorus distributes approximately 85% in bone, and the residual mainly in cells, and is a constituent of important substances such as energy-producing substances cell membranes, and DNA. Phosphorus is filtered through the kidneys and excreted in urine, but will remain in blood without being fully excreted under severe kidney dysfunction. It is therefore understood that the higher the blood phosphorus level, the lower the renal function.

Representative test items for determining liver function include alanine aminotransferase (ALT) and aspartate aminotransferase (AST). AST is also abundantly contained typically in heart muscle, skeletal muscle, red blood cell and the like, meanwhile ALT is mainly present in liver, and is therefore suitable for examining occurrence or degree of hepatocellular injury.

The pharmaceutical composition of the present invention has an effect of promoting lowering, maintenance, or suppression of elevation of at least one test value selected from serum creatinine (CRE), blood urea nitrogen (BUN), or blood phosphorus (IP). That is, the pharmaceutical composition of the present invention may be used for improving renal function. The pharmaceutical composition of the present invention may also be used for lowering, maintaining, or suppressing elevation of at least one test value selected from serum creatinine (CRE), blood urea nitrogen (BUN), or blood phosphorus (IP). In one preferred mode, the pharmaceutical composition has an effect of promoting lowering, maintenance, or suppression of elevation of all the test values regarding serum creatinine (CRE), blood urea nitrogen (BUN), and blood phosphorus (IP).

The pharmaceutical composition of the present invention also has an effect that administration thereof does not elevate the blood alanine aminotransferase (ALT) level. That is, the pharmaceutical composition of the present invention has a feature that administration thereof is less likely to induce decline of liver function.

Note in the present invention, the pharmaceutical composition used for improving renal function is preferably a therapeutic agent for kidney disease.

Note that the kidney disease in the present invention means declined state of renal function, typically due to kidney trouble such as acute kidney injury or chronic renal failure. Note also that the kidney disease in the context of the present invention encompasses not only disease relevant to declined renal function, but also disease of other organ associated to the declined renal function. The disease of other organ is exemplified by various diseases induced by uremic toxin, such as cardiac and vascular diseases.

The treatment of kidney disease in the context of the present invention encompasses treatment for various types of disorder caused by kidney disease, such as suppression and/or prevention of decline of renal function; maintenance of renal function; restoration or improvement of renal function; and prevention, suppression, improvement, or treatment of diseases associated to the declined renal function.

The pharmaceutical composition of the present invention may be used as a therapeutic agent for kidney disease. The kidney disease, to which the pharmaceutical composition of the present invention is applicable, is preferably exemplified by chronic kidney disease (CKD), acute kidney injury (AKI), and chronic renal failure. The pharmaceutical composition of the present invention may also be used for preventing or treating various diseases typically caused by excessive intake of phosphorus, such as hyperphosphatemia, renal failure, and osteoporosis.

The pharmaceutical composition of the present invention also has an action of lowering any substance that can accumulate in the body due to declined renal function. In the context of the present invention, any substance that can accumulate in the body due to declined renal function means any substance capable of inducing uremic symptom (uremic toxin), and/or uremic toxin precursor. That is, the pharmaceutical composition of the present invention may have a form of agent for lowering uremic toxin and/or precursor thereof, and more preferably a form of an agent for lowering uremic toxin.

The pharmaceutical composition of the present invention may therefore be used for treatment of uremia. The pharmaceutical composition of the present invention may be used for relieving or suppressing uremic symptom.

Note in the context of the present invention, uremia means systemic change that occurs as a result of extreme depression of renal function, which is observed in a progressed phase of acute or chronic kidney injury.

The present inventors have also found that cerium is absorbed into the body, by administering a cerium oxide dispersion in which cerium(III) ion as trivalent cerium and cerium oxide coexist, as described later in Examples. The present inventors have further confirmed that cerium is similarly absorbed in the body, more specifically accumulated in organs, also by administering an ionic compound that contains trivalent cerium, such as cerium chloride.

That is, the pharmaceutical composition of the present invention preferably demonstrates a desired effect in the form of cerium compound and/or cerium ion, as a result of bioabsorption of the cerium compound through the gastrointestinal tract.

In the context of the present invention, the gastrointestinal tract is preferably an intestinal tract.

In the present invention, gastrointestinal absorbability means that any substance such as active ingredient can be absorbed through the gastrointestinal tract into the body (mainly into blood).

The intestinal tract in the context of the present invention typically means small intestine (jejunum, ileum, duodenum, etc.), and large intestine (cecum, colon, rectum, etc.). The intestinal absorbability means that any substance such as active ingredient is absorbed through the intestinal tract into the body (mainly into blood).

The pharmaceutical composition of the present invention is preferably formulated in a gastrointestinally absorbable form. The dosage form in the present invention preferably allows cerium to exist in a trivalent form in the gastrointestinal tract. Processing form of the formulation is not particularly limited, and is suitably selectable typically from the processing forms of the aforementioned oral preparation.

In one preferred mode, the cerium compound according to the present invention is gastrointestinally absorbed, migrates into the blood, to reach various organs including kidneys, thereby demonstrating the operation and effect of the present invention.

The pharmaceutical composition of the present invention, if formulated in a gastrointestinally absorbable form, contains the cerium compound whose molecular weight is preferably 5,000 or smaller, more preferably 2,000 or smaller, and even more preferably 600 or smaller.

In the aforementioned mode, the present invention is also preferably embodied as a gastrointestinally absorbable pharmaceutical composition. The present invention may be embodied as an intestinally absorbable pharmaceutical composition.

The present invention may also be embodied in a mode where the cerium compound is gastrointestinally absorbable, and preferably intestinally absorbable. The present invention may also be embodied in a mode where the cerium compound which is gastrointestinally absorbable and more preferably intestinally absorbable, is administered.

In this mode, dosage form is preferably oral dose.

Note in this embodiment, the gastrointestinally absorbed cerium may exist in the form of monatomic ion, or in a combined form with other substance.

In the present invention, "absorption" through gastrointestinal tract or intestinal tract encompasses not only a mode where some component is taken into the body typically by active transport, but also a mode of passive transport where some component migrates into the body while driven typically by concentration.

The pharmaceutical composition, if in the gastrointestinally absorbable mode, preferably has a dosage form that keeps the cerium compound soluble in liquid, which is typically a dosage form that keeps cerium ionizable in the gastrointestinal tract. That is, the cerium compound in this mode may alternatively be in a mode where the cerium compound is not granulated, and/or, not processed for sustained release property or to be enteric, typically by coating.

The present inventors have also found that the blood uremic toxin level was lowered, by administering a cerium oxide dispersion in which cerium(III) ion and cerium oxide coexist. The present inventors have also confirmed that administration of an ionic cerium compound that contains trivalent cerium, such as cerium chloride, similarly lowered the blood uremic toxin level.

That is, the pharmaceutical composition of the present invention may be used for lowering, maintaining, or suppressing elevation of the uremic toxin level in the body, which is more specifically uremic toxin level in the blood.

An action mechanism by which the pharmaceutical composition of the present invention lowers the uremic toxin and/or the precursor thereof is considered to be a mechanism by which the cerium compound according to the present invention takes part in suppressing the uremic toxin and/or the precursor thereof from generating. A possible mode may be such that, for example, the cerium compound that contains trivalent cerium according to the present invention acts to decompose or detoxify the uremic toxin or the precursor thereof. The uremic toxin and/or the precursor thereof to be acted on are exemplified by tryptophan, kynurenine, serotonin, tryptan, indoles (indole acetic acid, indole propionic acid, indole lactic acid, indole, etc.), tyrosine, p-cresol, phenol, phosphatidylcholine, carnitine, trimethylamine (TMA), indoxyl, indoxyl sulfate, p-cresyl sulfate, phenyl sulfate, and trimethylamine-N-oxide (TMAO).

The uremic toxin to be reduced, maintained, or suppressed from elevating by the pharmaceutical composition of the present invention is preferably one species, or two or more species selected from indoxyl sulfate, p-cresyl sulfate, phenyl sulfate, and trimethylamine-N-oxide (TMAO).

The action mechanism, by which the pharmaceutical composition of the present invention lowers the uremic toxin and/or the precursor thereof, may be such that the cerium compound that contains trivalent cerium according to the present invention lowers activity of enzyme (inhibit enzyme) that metabolizes food-derived component to the uremic toxin precursor or the uremic toxin. The enzyme to be acted on is exemplified by tryptophan 2,3-dioxygenase (TDO), tryptophan hydroxylase (TPH), aromatic L-amino acid decarboxylase (AADC), tryptophanase, tyrosine phenol-lyase (β-tyrosinase), trimethylamine-lyase, hydroxylase, and sulfotransferase.

One embodiment of the present invention is exemplified by use of the cerium compound that contains trivalent cerium, for the manufacture of a pharmaceutical composition intended for improvement of renal function, preferably for treatment of kidney disease. Another possible mode may relate to use of the cerium compound that contains trivalent cerium, for the manufacture of a pharmaceutical composition intended for lowering, maintaining, or suppressing elevation of at least one test value selected from serum creatinine (CRE), blood urea nitrogen (BUN), and blood phosphorus (IP). Yet another possible mode may relate to use of the cerium compound that contains trivalent cerium, for the manufacture of a pharmaceutical composition intended for lowering, maintaining or suppressing elevation of uremic toxin level in the body, more preferably uremic toxin level in blood.

Yet another possible mode may relate to use of the cerium compound that contains trivalent cerium, for the manufacture of a pharmaceutical composition intended for treating, suppressing, or improving uremia, more specifically for lowering, maintaining, or suppressing elevation of uremic toxin level, preferably uremic toxin level in blood.

One embodiment of the present invention is exemplified by use of the cerium compound that contains trivalent cerium, as an active ingredient intended for improvement of renal function, preferably for treatment of kidney disease. Another possible mode may relate to use of the cerium compound that contains trivalent cerium, as an active ingredient intended for lowering, maintaining, or suppressing elevation of at least one test value selected from serum creatinine (CRE), blood urea nitrogen (BUN), and blood phosphorus (IP). Yet another possible mode may relate to use of the cerium compound that contains trivalent cerium, as an active ingredient intended for lowering, maintaining or suppressing elevation of uremic toxin level in the body, more preferably uremic toxin level in blood.

Yet another possible mode may relate to use of the cerium compound that contains trivalent cerium, as an active ingredient intended for treating, suppressing, or improving uremia, more specifically for lowering, maintaining, or suppressing elevation of uremic toxin level, preferably uremic toxin level in blood.

One embodiment of the present invention is also exemplified by the cerium compound that contains trivalent cerium, for use in improvement of renal function, preferably for treatment of kidney disease. Another possible mode may relate to the cerium compound that contains trivalent cerium, for lowering, maintaining, or suppressing elevation of at least one test value selected from serum creatinine (CRE), blood urea nitrogen (BUN), and blood phosphorus (IP). Yet another possible mode may relate to the cerium compound that contains trivalent cerium, for lowering, maintaining or suppressing elevation of uremic toxin level in the body, more preferably uremic toxin level in blood.

Yet another possible mode may relate to the cerium compound that contains trivalent cerium, as an active ingredient for treating, suppressing, or improving uremia, more specifically for lowering, maintaining, or suppressing elevation of uremic toxin level, preferably uremic toxin level in blood.

An embodiment of the present invention is exemplified by a therapeutic method for improvement of renal function, including administering the cerium compound that contains trivalent cerium to a subject in need of treatment for improvement of renal function. An embodiment of the present invention is also exemplified by a therapeutic method for kidney disease, including administering the cerium compound that contains trivalent cerium to a subject in need of treatment of kidney disease. Another possible mode may relate to a therapeutic method for kidney disease, including administering the cerium compound that contains trivalent cerium, to a subject in need of treatment for lowering, maintaining, or suppressing elevation of at least one test value selected from serum creatinine (CRE), blood urea nitrogen (BUN), and blood phosphorus (IP). Yet another possible mode may relate to a therapeutic method for kidney disease, including administering the cerium compound that contains trivalent cerium, to a subject in need of treatment for lowering, maintaining, or suppressing elevation of uremic toxin level in the body, more preferably uremic toxin level in blood.

Yet another possible mode may relate to a therapeutic method for uremia, including administering the cerium compound that contains trivalent cerium, to a subject in need of treatment, suppression, or improvement of uremia. Yet another possible mode may relate to a method for lowering, maintaining, or suppressing elevation of the uremic toxin level, wherein the method includes administering the cerium compound that contains trivalent cerium to a subject in need of lowering, maintaining or suppressing elevation of uremic toxin level, preferably uremic toxin level in blood.

For the evaluation of the pharmaceutical composition of the present invention, an adenine nephropathy model may be used as a chronic renal failure model.

The adenine nephropathy model in rats is created by keeping the rats under dietary load with adenine to induce chronic progressive renal disorder, and is considered to be a good model of human chronic kidney disease (CKD) patient obtainable only with the adenine load.

### Examples

### <Test Example 1> Test for confirming effectiveness of cerium compound that contains trivalent cerium

### (Example 1)

The adenine nephropathy model rats were fed with a dispersion that contains cerium(III) ion as the trivalent cerium, and cerium oxide, to confirm the effect of cerium compound that contains trivalent cerium, according to the pharmaceutical composition of the present invention.

Materials, feeds, and equipment used for animal experiments are listed below.

### [Materials, Feeds, Equipment, etc.]

· Rat: Slc:Wistar rat (male) delivered at 12 weeks of age (Japan SLC, Inc.)
· Powder feed: AIN-93G powder feed (Funabashi Farm Co., Ltd.)
· Feed for laboratory animal: Labo MR Stock (Nosan Corporation)
· Adenine: (Tokyo Chemical Industry Co., Ltd.)
· Cerium oxide dispersion: Cerium oxide dispersion prepared in [Preparation of Cerium Oxide Dispersion] (10.0% by mass aqueous cerium oxide dispersion, with mole ratio of cerium oxide:cerium(III) ion = 9:1)
· Cerium(III) chloride: cerium(III) chloride heptahydrate (Nikki Corporation)
· Water or hot water: water allowed to pass through a water purifier, and then boiled in a pot
· Mixer for feed preparation: Compact kitchen machine MUM4415JP (Bosch GmbH)
· Manual sausage maker: sausage machine (COM4SPORT)
· Wing blood collection needle for animal experiment: CL-4597 (CLEA Japan, Inc.)

### [Preparation of Cerium Oxide Dispersion]

In a reaction vessel equipped with a stirrer, placed were 469.0 g of water, 229.072 g of cerium(III) chloride heptahydrate (Nikki Corporation), and 5.556 g of sodium acetate (FUJIFILM Wako Pure Chemical Corporation), to prepare an aqueous solution with a cerium concentration of 1.15 mol/L, which was kept stirred at 20°C.

To the solution, 14.629 g of a 35.4% by mass hydrogen peroxide solution (FUJIFILM Wako Pure Chemical Corporation) was added, and the content was kept stirred for 5 minutes.

To the solution, 350.542 g of a 4 mol/L aqueous sodium hydroxide solution (FUJIFILM Wako Pure Chemical Corporation) was further added, and the content was kept stirred for 5 minutes.

The solution was then heated to 45°C over 10 minutes, kept stirred for 60 minutes, and then cooled down to room temperature.

The final cerium concentration of the solution was found to be 0.7 mol/L.

Thereafter, the reaction solution was washed with water, filtered, and desalted to prepare an aqueous dispersion having a cerium oxide concentration of 10.0% by mass, and a molar ratio of cerium oxide and cerium(III) ion in the solution of 9:1 (cerium oxide dispersion, also simply referred to as dispersion, hereinafter). The molar ratio of cerium oxide and cerium(III) ion may be measured by inductively coupled plasma mass spectrometry (ICP-MS), or inductively coupled plasma atomic emission spectrometry (ICP-AES).

From observation of the obtained particle under a transmission electron microscope (TEM), cerium oxide was found to have an average particle size of 4.0 nm.

### [Preparation of Feeds]

### (1) Normal Group Feed

(i) Weighed was 250 g of a powder feed, which was then kneaded with a kitchen machine.
(ii) After kneaded with a kitchen machine for 3 minutes, the feed adhered on the wall surface was scraped off, and then kneaded again for 3 minutes. Approximately 100 mL of hot water was then added evenly.
(iii) The feed was once put together by hands, and further kneaded with the kitchen machine until it became homogeneous.
(iv) The thus kneaded feed was shaped into a rod through a sausage machine.
(v) The thus shaped feed was cut into a cylindrical shape approximately 2.5 cm in diameter and 1.5 cm in thickness.
(vi) The thus shaped cylindrical feed was dried at 38°C in an incubator for two days.
(vii) The feed was weighed to estimate a proportion of residual water content, and dried until the proportion drops to 10% or below.
(viii) The prepared feed was put in a zipper bag, and stored in a refrigerator.

The special feed was thus prepared by repeating steps (i) to (viii) necessary number of times.

### (2) Adenine Group Feed

A special feed was prepared by modifying step (ii) for the normal group feed, as described below.
(ii) (Ade): While keeping the content under kneading, 0.625 g of adenine was added in small portions, the content was kneaded with a kitchen machine for 3 minutes, the feed adhered on the wall surface was scraped off, and the content was kneaded again for 3 minutes. Approximately 100 mL of hot water was then added evenly.

### (3-1) Adenine + Cerium Oxide Dispersion (A) Group Feed

A special feed was prepared by modifying steps (i) and (ii) for (2) Adenine Group Feed, as described below.
(i) (Ade + Dispersion (A)): Weighed was 246.1 g of the powder feed, which was then kneaded with a kitchen machine.
(ii) (Ade + Dispersion (A)): While keeping the content under kneading, 0.625 g of adenine was added in small portions, the content was kneaded with the kitchen machine for 3 minutes, the feed adhered on the wall surface was scraped off, and the content was kneaded again for 3 minutes. Next, while keeping the content under kneading, 32.7 g of cerium oxide dispersion was added in small portions, the content was kneaded with the kitchen machine for 3 minutes, the feed adhered on the wall surface was scraped off, and the content was kneaded again for 3 minutes. Approximately 70 mL of hot water was then added evenly.

### (3-2) Adenine + Cerium Oxide Dispersion (B) Group Feed

A special feed was prepared by modifying steps (i) and (ii) for the adenine group feed, as described below.
(i) (Ade + Dispersion (B)): Weighed was 240.1 g of the powder feed, which was then kneaded with the kitchen machine.
(ii) (Ade + Dispersion (B)): While keeping the content under kneading, 0.625 g of adenine was added in small portions, the content was kneaded with the kitchen machine for 3 minutes, the feed adhered on the wall surface was scraped off, and the content was kneaded again for 3 minutes. Next, while keeping the content under kneading, 98.2 g of cerium oxide dispersion was added in small portions, the content was kneaded with the kitchen machine for 3 minutes, the feed adhered on the wall surface was scraped off, and the content was kneaded again for 3 minutes. Approximately 12 mL of hot water was then added evenly.

### [Breeding of Rats]

(1) Rats were weighed, and classified into four groups.
(2) Each group was acclimatized with Labo MR Stock feed, under ad libitum feeding and ad libitum water drinking. The feeding cage was conditioned at room temperature 22 ± 2°C, under illumination 12 hours a day.
(3) After acclimatized, the animals were bred with any of the special feeds under ad libitum feeding and ad libitum water drinking. Feeding cages were conditioned in the same way as in the acclimatization period, at room temperature 22 ± 2°C, under illumination 12 hours a day.
(4) On Days 0, 7, 14, 21, and 28, rats were bred in blood collection/metabolic cages for 24 hours.
   The animals were fed with Labo MR Stock on Day 0, and with the special feed on days thereafter, during which the amounts of feed consumption, water consumption, urine and feces were measured, and the urine and feces were sampled.
(5) The animals were taken out of the metabolic cages and weighed, followed by blood collection with a winged needle. Approximately 500 µL of blood was collected, the blood was incubated at room temperature for one hour, and then centrifuged (1,700 × G, 20 min) to obtain serum.
   Thereafter, (4) breeding in the metabolic cages, and (5) weight measurement and blood collection, were repeated.
(6) On the final Day 28, the animals were fasted throughout the day, and then dissected. The animals were subjected to laparotomy under anesthesia, and exsanguinated to death by collecting blood through the inferior vena cava. The obtained blood was incubated at room temperature for one hour, and then centrifuged (1,700 × G, 20 min) to obtain serum. Also heparinized whole blood was collected, and centrifuged (1,700 × G, 20 min) to obtain plasma.

Liver, kidneys, spleen, small intestine, and stomach of the rats sacrificed by exsanguination were collected. The small intestine and stomach were dissected, removed the contents, and rinsed twice with PBS.

Analysis of the collected blood sample was outsourced to Oriental Yeast Co., Ltd.

Analytical methods and analytical reagent for the individual test items will be listed below.

### [Analyses of Blood Samples]

· Serum creatinine (CRE): enzymatic method, L-type Wako CRE·M (FUJIFILM Wako Pure Chemical Corporation)
· Blood urea nitrogen (BUN): urease-GLDH method, reagent from Oriental Yeast Co., Ltd.
· Serum phosphorus (IP): enzymatic method, Determiner L IP II (Hitachi Chemical Diagnostics Systems Co., Ltd.)
· Alanine aminotransferase (ALT): JSCC transferable method, L-type Wako ALT·J2 (FUJIFILM Wako Pure Chemical Corporation)
· Serum albumin (ALB): BCG method, Albumin II HA-Test Wako (FUJIFILM Wako Pure Chemical Corporation)

These items were measured with use of Hitachi 7180 automatic analyzer.

Figs. 1 to 4 illustrate diagrams plotting test values of serum creatinine (CRE), blood urea nitrogen (BUN), serum phosphorus (IP), or alanine aminotransferase (ALT) of the blood samples collected by the dissection on the ordinates, and plotting total molar amount of cerium contained in 100 g of feed on the abscissae.

The adenine group fed under the adenine group feed (total cerium concentration = 0 mmol/100 g) was found to demonstrate elevated test values for CRE, BUN and IP, as compared with the normal group fed under the normal group feed, indicating declined renal function, that is, onset of adenine nephropathy. ALT was found to be equivalent to that in the normal group.

The values for CRE, BUN, and IP were found to decline as the cerium oxide content in the feed increased, proving that the degree of improvement in renal function tends to increase. ALT was found to largely vary at high content of cerium oxide, meanwhile remained almost unchanged up to or around a total cerium concentration of 10 mmol/100 g.

From the results above, the dispersion liquid that contains cerium(III) ion, which represents the trivalent cerium compound, and cerium oxide was confirmed to be effective for assisting, maintaining, or improving the renal function.

### (Example 2)

The adenine nephropathy model rats were fed with cerium(III) chloride heptahydrate, as the cerium compound that contains trivalent cerium, to examine effect of the cerium compound that contains trivalent cerium, according to the pharmaceutical composition of the present invention.

The effect of cerium(III) chloride was examined with use of the following feeds (4-1) to (4-3), in place of the (3-1) Adenine + Cerium Oxide Dispersion Liquid (A) Group Feed, and the (3-2) Adenine + Cerium Oxide Dispersion Liquid (B) Group Feed, having been described in [Preparation of Feeds] according to Example 1.

### (4-1) Adenine + Cerium Chloride (C) Group Feed

A special feed was prepared as described below, by modifying steps (i) and (ii) (Ade) for the adenine group feed.
(i) (Ade + CeCl₃ (C)): Weighed was 248.8 g of the powder feed, which was then kneaded with the kitchen machine.
(ii) (Ade + CeCl₃ (C)): While kneading the content, 0.625 g of adenine was added in small portions, the content was kneaded with the kitchen machine for 3 minutes, the feed adhered on the wall surface was scraped off, and the content was kneaded again for 3 minutes. Next, while keeping the content under kneading, 10 mL of aqueous solution that contains 1.79 g of cerium(III) chloride heptahydrate dissolved therein was added in small portions, the content was kneaded with the kitchen machine for 3 minutes, the feed adhered on the wall surface was scraped off, and the content was kneaded again for 3 minutes. Approximately 90 mL of hot water was then added evenly.

### (4-2) Adenine + Cerium Chloride (D) Group Feed

A special feed was prepared as described below, by modifying steps (i) and (ii) (Ade) for the adenine group feed.
(i) (Ade + CeCl₃ (D)): Weighed was 247.7 g of the powder feed, which was then kneaded with the kitchen machine.
(ii) (Ade + CeCl₃ (D)): While kneading the content, 0.625 g of adenine was added in small portions, the content was kneaded with the kitchen machine for 3 minutes, the feed adhered on the wall surface was scraped off, and the content was kneaded again for 3 minutes. Next, while keeping the content under kneading, 20 mL of aqueous solution that contains 3.58 g of cerium(III) chloride heptahydrate dissolved therein was added in small portions, the content was kneaded with the kitchen machine for 3 minutes, the feed adhered on the wall surface was scraped off, and the content was then kneaded again for 3 minutes. Approximately 80 mL of hot water was then added evenly.

### (4-3) Adenine + Cerium Chloride (E) Group Feed

A special feed was prepared as described below, by modifying steps (i) and (ii) (Ade) for the adenine group feed.
(i) (Ade + CeCl₃ (E)): Weighed was 245.3 g of the powder feed, which was then kneaded with the kitchen machine.
(ii) (Ade + CeCl₃ (E)): While kneading the feed, 0.625 g of adenine was added in small portions, the content was kneaded with the kitchen machine for 3 minutes, the feed adhered on the wall surface was scraped off, and the content was kneaded again for 3 minutes. Next, while keeping the content under kneading, 40 mL of aqueous solution that contains 7.17 g of cerium(III) chloride heptahydrate dissolved therein was added in small portions, the content was kneaded with the kitchen machine for 3 minutes, the feed adhered on the wall surface was scraped off, and the content was kneaded again for 3 minutes. Approximately 60 mL of hot water was then added evenly.

Figs. 5 to 8 respectively illustrate diagrams plotting test values of serum creatinine (CRE), blood urea nitrogen (BUN), serum phosphorus (IP), or alanine aminotransferase (ALT) of the blood samples collected by the dissection on the ordinates, and plotting the molar amount of cerium chloride contained in 100 g of feed on the abscissae.

Similarly to as in Example 1, the adenine group fed under the adenine group feed (cerium chloride concentration = 0 mmol/100 g) was found to demonstrate elevated test values for CRE, BUN and IP, as compared with the normal group fed under the normal group feed, indicating declined renal function, that is, onset of adenine nephropathy. ALT was found to be equivalent to that in the normal group.

The values for CRE, BUN, and IP were found to decline as the cerium chloride content in the feed increased, proving that the degree of improvement in renal function tends to increase. ALT was found to be equivalent to that in the normal group.

For better understanding of differences in the effects between the cerium oxide dispersion and cerium chloride, Figs. 9 to 12 illustrate test values for the feed that contains cerium oxide dispersion, and test values for the feed that contains cerium chloride, normalized while scaling an averaged test value in each adenine feed group at 100.

The cerium chloride-containing feed was found to decrease test values of all of CRE, BUN, and IP, with a smaller molar amount of cerium, as compared with the cerium oxide dispersion-containing feed. Use of cerium chloride was estimated to yield the effects approximately 4 to 9 times larger than that obtainable from the cerium oxide dispersion. On the other hand, ALT level was found to be equivalent to that in the normal group, in both cases of using cerium chloride and the cerium oxide dispersion.

The results revealed that cerium(III) chloride heptahydrate, used as an example of the cerium compound that contains trivalent cerium according to the pharmaceutical composition of the present invention, has an effect of assisting, maintaining or improving the renal function. Cerium(III) chloride heptahydrate was also suggested to have larger potential typically in assisting the renal function, than the cerium oxide dispersion.

The pharmaceutical product of the present invention having, as an active ingredient, the cerium compound that contains trivalent cerium, may therefore be usable for lowering, maintaining, or suppressing elevation of the test values of serum creatinine (CRE), blood urea nitrogen (BUN), or serum phosphorus (IP), that is, may be applicable to improve renal function, or as kidney disease treatment drug.

### <Test Example 2> Evaluation of in-vivo Absorbability of Cerium in Cerium Oxide Dispersion

Healthy rats were fed on the cerium oxide dispersion, to evaluate the permeability into the blood. Explanations on the materials, feeds, equipment and so forth used in the animal experiments, similar to those in Test Example 1, will be appropriately skipped.

### [Preparation of Cerium Oxide Dispersion]

The cerium oxide dispersion was prepared according to the same procedures as in Test Example 1.

### [Preparation of Feeds]

### (1) Normal Group Feed

Rats for the normal group were bred according to the same procedures as in Test Example 1.

### (2) Cerium Oxide Dispersion Group Feed

A special feed was prepared as described below, by modifying steps (i) and (ii) for the normal group in Test Example 1.
(i) (Dispersion): Weighed was 246.7 g of the powder feed, which was then kneaded with the kitchen machine.
(ii) (Dispersion): The content was kneaded with the kitchen machine for 3 minutes, the feed adhered on the wall surface was scraped off, and the content was kneaded again for 3 minutes. Next, while keeping the content under kneading, 32.7 g of cerium oxide dispersion was added in small portions, the content was kneaded with the kitchen machine for 3 minutes, the feed adhered on the wall surface was scraped off, and the content was kneaded again for 3 minutes. Approximately 70 mL of hot water was then added evenly.

### [Breeding of Rats]

The test was conducted according to the same procedures as in Test Example 1, except that the rats were weighed and divided into two groups.

### [Analysis of Cerium Content in Blood and Organ Samples]

The blood, liver, and kidneys collected from the cerium oxide dispersion group were treated by nitric acid dissolution and aqua regia dissolution to prepare sample solutions, and measured by inductively coupled plasma mass spectrometry (Agilent 7900 ICP-MS, from Agilent Technologies).

The measurement revealed the cerium contents per one gram of blood and of the individual organs, as listed below.
· Blood < 0.0020 µg Ce/g blood
· Liver 0.71 µg Ce/g liver
· Kidney 0.29 µg Ce/g kidney

The results revealed that only a slight amount of cerium was retained in blood, meanwhile the cerium content was found to be high in liver followed by kidneys, per unit mass of organ. Cerium, ingested in the form of cerium oxide dispersion, is therefore considered to be absorbed through the digestive tract into the body.

### <Test Example 3> Uremic Toxin Lowering Action by Cerium Oxide Dispersion

The adenine nephropathy model rats, to which the cerium oxide dispersion used in Example 1 was administered, were subjected to the steps up to [Breeding of Rats] in the same way as in Example 1, and the acquired plasma was subjected to measurement of the plasma uremic toxins (indoxyl sulfate, p-cresyl sulfate, phenyl sulfate, and trimethylamine-N-oxide (TMAO)).

Analyses of indoxyl sulfate, p-cresyl sulfate, phenyl sulfate, and trimethylamine-N-oxide (TMAO) were outsourced to LSI Medience Corporation.

Analyzers used in the individual test items are listed below.
· Indoxyl sulfate, p-cresyl sulfate, phenyl sulfate: LCMS-8050, from Shimadzu Corporation
· Trimethylamine-N-oxide (TMAO): Agilent 6545 Q-TOF LC/MS, from Agilent Technologies, Inc.

Figs. 13 to 16 respectively illustrate diagrams plotting plasma levels of indoxyl sulfate, p-cresyl sulfate, phenyl sulfate, and trimethylamine-N-oxide (TMAO) in the blood samples collected by the dissection on the ordinates, and plotting total molar amount of cerium contained in 100 g of feed on the abscissae.

The adenine group fed with the adenine group feed (total cerium concentration = 0 mmol/100 g) was found to demonstrate elevated levels of all of indoxyl sulfate, p-cresyl sulfate, phenyl sulfate, and trimethylamine-N-oxide (TMAO), as compared with the normal group fed under the normal group feed.

The levels of all of indoxyl sulfate, p-cresyl sulfate, phenyl sulfate, and trimethylamine-N-oxide (TMAO) were found to demonstrate declining tendency, as the total cerium content in the feed increased.

Judging from the above, the cerium oxide dispersion was confirmed to be effective in lowering the blood uremic toxin. The results teach that the pharmaceutical composition of the present invention may be used for lowering, maintaining or suppressing elevation of blood uremic toxin, that is, applicable as a uremic toxin lowering agent. Cerium oxide and/or trivalent cerium ion, contained in the cerium oxide dispersion, are now judged to contribute typically to lowering in blood uremic toxin.

The results of Test Examples 2 and 3 also teach that cerium contained in the cerium oxide dispersion presumably reduces the uremic toxin level in blood of the subject to which the cerium oxide dispersion was administered, as a result of bioabsorption.

### <Test Example 4> Evaluation of in vivo Cerium Absorption with Use of Cerium Chloride

The adenine nephropathy model rats used in the aforementioned Example 2, to which cerium chloride has been administered, were subjected to the steps up to [Breeding of Rats] in the same way as in Example 2, and then evaluated for bioabsorbability of cerium chloride.

The specific procedures are same as those in the analysis of cerium content in the organ samples in Test Example 2.

The measurement revealed the cerium contents per one gram of blood and the individual organs, as listed below (see also Figs. 17 and 18).
(1) Under cerium chloride-mixed feed with the total cerium content equivalent to 1.9 mmol/100 g
   · Liver 2.04 µg Ce/g liver
   · Kidney 0.35 µg Ce/g kidney
(2) Under cerium chloride-mixed feed with the total cerium content equivalent to 3.9 mmol/100 g
   · Liver: 4.14 µg Ce/g liver
   · Kidney: 1.25 µg Ce/g kidney

The results revealed that, after administered in the form of cerium chloride, cerium was absorbed into the body, and transferred to liver and kidneys. The results also revealed that the cerium content was high in liver, followed by kidneys, and that the cerium level in the organs increased depending on the cerium chloride concentration. The results imply that, under the cerium chloride feed, cerium was absorbed through the gastrointestinal tract (such as intestinal tract) into the body.

The results also revealed that feeding on cerium chloride resulted in larger amounts of cerium accumulation in the organs, as compared with Test Example 2 (under cerium oxide dispersion-mixed feed with the total cerium content adjusted to 72.2 mmol/100g feed). The results teach that the ionic compound that contains trivalent cerium, such as cerium chloride, causes larger amounts of bioabsorption of cerium, as compared with cerium oxide.

### <Test Example 5> Uremic Toxin Lowering Action by Cerium Chloride

The adenine nephropathy model rats used in the aforementioned Example 2, to which cerium(III) chloride heptahydrate has been administered, were subjected to the steps up to [Breeding of Rats] in the same way as in Example 2, and the acquired whole blood was analyzed for the uremic toxin (indoxyl sulfate) level in whole blood.

Specific procedures are as follows.

### [Deproteinization of Whole Blood]

A solvent for precipitation (5.56 µmol/L 2-naphthalenesulfonic acid solution in acetonitrile) and a blood sample (whole blood) were mixed in a 1.5 mL polypropylene tube, the mixture was allowed to stand still at room temperature, and then centrifuged to cause solid-liquid separation, thereby obtaining a supernatant. A 10 mmol/L aqueous ammonium acetate solution was added to the thus obtained supernatant, and the mixture was centrifuged to obtain a supernatant. The operation above diluted the blood sample 50 times, with the final concentration of the thus-added 2-naphthalenesulfonic acid adjusted to 1 µmol/L.

### [HPLC Measurement]

With use of an autosampler, 2.5 µL of the thus-obtained blood sample was injected, and a component that demonstrates absorption at 280 nm was detected with a UV-visible (UV-VIS) spectroscopic detector, under the measurement conditions below.
· Column: CEPCELL PAK MADE HR S5 (2.1 × 150 mm, Osaka Soda Co., Ltd.)
· Column oven temperature: 40°C
· Mobile phase: 10 mol/L ammonium acetate/acetonitrile = 80/20
· Flow rate: 100 µL/min

Fig. 19 illustrates a diagram, with the indoxyl sulfate concentration in the blood sample collected by dissection plotted on the ordinate, and with the total mole amount of cerium contained in 100 g of feed plotted on the abscissa. The adenine group fed under the adenine group feed (total cerium concentration = 0 mmol/100 g) was found to demonstrate elevated levels of indoxyl sulfate, as compared with the normal group fed under the normal group feed. Moreover, under feed of adenine combined with cerium chloride, indoxyl sulfate concentration was found to demonstrate a declining tendency.

The results above teach that the pharmaceutical composition of the present invention, having the ionic compound that contains trivalent cerium such as cerium chloride, is considered to be applicable to lowering, maintenance or suppression of elevation of blood uremic toxin, that is, applicable as a uremic toxin lowering agent.

### <Test Example 6> Test for Confirming Solubility of Cerium Compound in Simulated Gastric Fluid

Among trivalent ceriums related to the pharmaceutical composition of the present invention, cerium chloride, cerium nitrate, and cerium acetate, which are ionic compounds, were examined for their solubility in a solution that simulates gastric fluid (simulated gastric fluid). The simulated gastric fluid used herein was the 1st fluid for disintegration test specified for the dissolution test in the Japanese Pharmacopoeia, prepared by a method in compliance with the pharmacopoeia. Specific procedures are as follows.

### [Materials, Equipment, etc.]

· Cerium(III) chloride: Nikki Corporation
· Cerium(III) nitrate: Nikki Corporation
· Cerium(III) acetate: Nikki Corporation
· Cerium oxide dispersion: prepared by the same procedures as in Test Example 1
· Sodium chloride: Tomita Pharmaceutical Co., Ltd.
· Hydrochloric acid (35.0 to 37.0%): Kanto Chemical. Co., Inc.
· Benchtop pH meter: Horiba, Ltd.
· Volumetric flask (10 mL, 100 mL): SIBATA Scientific Technology, Ltd.
· Quartz cell for spectrophotometer: (Optical path length: 10 mm, optical path width: 10 mm): AS ONE Corporation
· Absorptiometer: A & E La (UK) Co., Ltd.

### [Preparation of Simulated Gastric Fluid]

In a volumetric flask (100 mL), placed were 50 mL of purified water and 8.26 g of hydrochloric acid, to which 2.00 g of sodium chloride was added. Purified water was filled up to the marked line of the volumetric flask, and the content was stirred thoroughly, to prepare 100 mL in total of aqueous solution. The aqueous solution was diluted 10 times to prepare a simulated gastric fluid (1000 mL, pH: ca. 1.2), which corresponds to the disintegration test liquid 1.

### [Preparation of Measurement Sample]

Cerium chloride, cerium nitrate, or cerium acetate was dissolved in the simulated gastric fluid, so as to adjust each concentration to 0.05 M. More specifically, the simulated gastric fluid was added to each of 0.19 g of cerium chloride, 0.22 g of cerium nitrate, or 0.18 g of cerium acetate, up to 10 mL in total. The obtained mixtures were stirred by inversion mixing at room temperature for about one hour, for use as measurement samples.

Also Comparative Example was conducted, in which the cerium oxide dispersion was added to the simulated gastric fluid. In Comparative Example, first, the cerium oxide dispersion was diluted with the simulated gastric fluid so as to adjust the total cerium concentration in fluid to 0.05 M. More specifically, the simulated gastric fluid was added to 0.86 g of the cerium oxide dispersion, up to 10 mL in total. The obtained mixtures were stirred by inversion mixing at room temperature for about one hour, for use as measurement samples.

### [Confirmation of Absorbance]

Each measurement sample was placed in a quartz cell of a spectrophotometer, and the absorbance at 200 to 600 nm was measured with an absorptiometer.

Figs. 20 and 21 illustrate appearance of the measurement samples, and the measurement results of the absorbance of the measurement samples, respectively. All measurement samples, prepared by adding cerium chloride, cerium nitrate, or cerium acetate to the simulated gastric fluid, were clear liquids without visually observable precipitate. Measurement of absorbance of the individual measurement samples demonstrated specific absorption in the ultraviolet region from approximately 200 to 340 nm, all without demonstrating absorbance at 340 nm or longer wavelength region.

This revealed that cerium chloride, cerium nitrate, and cerium acetate can dissolve into gastric fluid or the like, with similar solubility.

On the other hand, the measurement sample obtained by adding the cerium oxide dispersion to the simulated gastric fluid appeared to be a fluorescent yellow liquid, without visually observable precipitate. Measurement of absorbance of the measurement sample demonstrated absorption in a broad range that covers 200 to 600 nm.

This revealed that the cerium oxide dispersion demonstrates solubility different from those of the ionic compounds that contain trivalent cerium, such as cerium chloride.

The results above suggested that cerium(III) chloride, cerium(III) nitrate, and cerium(III) acetate can dissolve into gastric fluid, with similar solubility. In contrast, the cerium particle contained in the cerium oxide dispersion was found to be dispersed in a particle form, without being fully dissolved even in a strongly acidic solution like the simulated gastric fluid.

The results indicate that, from among cerium compounds, the ionic compound that contains trivalent cerium, such as cerium chloride, can dissolve in the gastrointestinal tract, typically in stomach, and can exist in the form of cerium ion. Additionally taking the test results on bioabsorbability in Test Example 4 into account, all of the ionic compounds that contain trivalent cerium are considered to express the effect after absorbed into the body, unlike the cerium oxide that forms a particle having a certain size.

Judging from the above, cerium chloride, cerium nitrate, or cerium acetate, when administered orally for example, are considered to express operations and effects as a result of absorption of cerium ion in some certain mode in the gastrointestinal tract.

The results of Test Examples 4 to 6 also teach that the ionic compound that contains trivalent cerium presumably lowers the blood uremic toxin level of the subject who have received the ionic compound, as a result of bioabsorption of cerium through the gastrointestinal tract.

### Industrial Applicability

The present invention may be used for improvement of renal function, more specifically, treatment, prevention, and/or suppression of progress of kidney disease, and secondary diseases associated therewith.

## Claims

1. A pharmaceutical composition having, as an active ingredient, a cerium compound that contains trivalent cerium.

2. The pharmaceutical composition according to claim **1,** wherein the cerium compound is an ionic compound.

3. The pharmaceutical composition according to claim 1 **or 2,** intended for use in improvement of renal function, and/or treatment, suppression or improvement of uremia.

4. The pharmaceutical composition according to any one of claims 1 to 3, wherein the cerium compound is any one of, or two or more species selected from cerium chloride, cerium acetate, cerium nitrate, cerium citrate, and hydrates of these compounds.

5. The pharmaceutical composition according to any one of claims 1 to 4, containing cerium oxide.

6. The pharmaceutical composition according to any one of claims 1 to 5, intended for use in lowering, maintenance, or suppression of elevation of at least one test value selected from serum creatinine (CRE), blood urea nitrogen (BUN), or blood phosphorus (IP).

7. The pharmaceutical composition according to any one of claims 1 to 6, intended for use in lowering, maintenance, or suppression of elevation of uremic toxin level.

8. The pharmaceutical composition according to claim 7, wherein the uremic toxin is blood uremic toxin.

9. The pharmaceutical composition according to claim 7 or 8, wherein the uremic toxin is any one of, or two or more species selected from indoxyl sulfate, p-cresyl sulfate, phenyl sulfate, and trimethylamine-N-oxide (TMAO).

10. The pharmaceutical composition according to any one of claims 1 to 9, being gastrointestinally absorbable.
